**Europäisches Patentamt**

**European Patent Office**

**Office européen des brevets**

(19)

(11) Veröffentlichungsnummer: **0 287 763 B1**

(12) # EUROPÄISCHE PATENTSCHRIFT

(45) Veröffentlichungstag der Patentschrift: **30.12.92**

(21) Anmeldenummer: **88102156.2**

(22) Anmeldetag: **13.02.88**

(51) Int. Cl.⁵: **A61B 17/02**, A61M 25/00, A61M 29/00

(54) **Vorrichtung zum Offenhalten eines Ureterotomieschnitts.**

(30) Priorität: **16.04.87 DE 8705674 U**

(43) Veröffentlichungstag der Anmeldung:
**26.10.88 Patentblatt 88/43**

(45) Bekanntmachung des Hinweises auf die
Patenterteilung:
**30.12.92 Patentblatt 92/53**

(84) Benannte Vertragsstaaten:
**AT BE CH DE ES FR GB IT LI NL**

(56) Entgegenhaltungen:
DE-A- 3 517 813
FR-A- 2 561 107
GB-A- 2 187 390
US-A- 4 643 716

(73) Patentinhaber: **ANGIOMED Aktiengesellschaft
Eisenbahnstrasse 36
W-7500 Karlsruhe 41(DE)**

(72) Erfinder: **Schnepp-Pesch, Wolfram
Schönblick 6
W-7505 Ettlingen(DE)**
Erfinder: **Lindenberg, Josef
Käthe-Kollwitz-Strasse 10a
W-7500 Karlsruhe 1(DE)**

(74) Vertreter: **Dr.-Ing. Hans Lichti Dipl.-Ing. Heiner
Lichti Dipl.-Phys. Dr. Jost Lempert
Postfach 41 07 60 Bergwaldstrasse 1
W-7500 Karlsruhe 41(DE)**

EP 0 287 763 B1

## Beschreibung

Die Erfindung betrifft eine Vorrichtung zum Offenhalten eines Ureterotomieschnitts im Harnleiter, mit einer im wesentlichen gestreckten Ureterotomieschiene.

Bei der Ureterotomie handelt es sich um eine operative Längsspaltung des Harnleiters, insbesondere zur Behebung einer Stenose, aber auch zur Weiterung zwecks Extraktion von Steinen. Bisher wurden Ureterotomien in der Regel transkutan von der Niere her durchgeführt, wobei dann auch in gleicher Weise eine transrenale Schienung vorgenommen wurde. Auf diese Weise konnten nur Stenosen im oberen Bereich des Harnleiters behandelt werden, so daß die entsprechenden Schienen von der Niere her nur ein Stück in den Harnleiter hineinragten, um den im oberen Bereich desselben befindlichen Ureterotomieschnitts offen zu halten. Sie wiesen daher eine Offenhaltung ausreichende entsprechende Dicke über ihre gesamte Länge auf, lediglich das in den Harnleiter ragende Ende war etwas verjüngt. Die Schienung dient dazu ein Zusammenwachsen und das Bilden von Verwachsungen im Bereich des Schnittes zu verhindern, was nur noch zu stärkeren Stenosen führen würde.

Neuerdings ist es möglich, die Ureterotomie auch von der Harnblase her vorzunehmen. Damit wurde die Möglichkeit geschaffen, Stenosen im gesamten Bereich des Harnleiters durch Ureterotomie zu behandeln. Es ist daher notwendig, die entsprechenden Schnitte, die sich nun an jeder beliebigen Stelle des Harnleiters befinden können offen zu halten.

Der Erfindung liegt daher die Aufgabe zugrunde, eine gattungsgemäße Vorrichtung zu schaffen, die zur Offenhaltung von Ureterotomieschnitten im gesamten Bereich des Harnleiters einsetzbar ist, dabei aber einen guten Flüssigkeitsabfluß gewährleistet.

Erfindungsgemäß wird die genannte Aufgabe bei einer Vorrichtung zum Offenhalten eines Ureterotomieschnitts im Harnleiter, mit einer im wesentlichen gestreckten Ureterotomieschiene (wie z.B. aus DE-A-3 517 813 bekannt) dadurch gelöst, daß auf der Ureterotomieschiene ein Aufweitkörper verschiebbar aufsitzt.

Naheliegend wäre es möglicherweise gewesen, die Schiene über ihre gesamte Länge hin derart stark auszubilden, daß sie den Ureterotomieschnitt zuverlässig aufhalten kann, unabhängig davon, an welcher Stelle im Katheter sich die Stenose befand. Es hat sich aber herausgetellt, daß dicke Schienen, auch wenn sie selbst ein großes Lumen aufweisen, durch welches ansich die Flüssigkeit aus der Niere ablaufen könnte, die Eigenperistaltik der Niere und des Harnleiters verhindern, so daß die Flüssigkeit schlechter abläft als bei einer dünneren Schiene mit geringerem Lumen. Dies liegt daran, daß diese die Eigenperistaltik nicht hindert und daher die Flüssigkeit aktiv auch an der Außenseite der Schiene vorbeigefördert werden kann. Eine dünnere Schiene hat daher eine größere Drainagewirkung als dicke Schienen. Andererseits muß aber die Schiene zumindestens im Bereich der aufgeschnittenen Stenose eine hinreichende Stärke aufweisen, um ein erneutes Zusammen- und Verwachsen des Schnittes zu verhindern. Durch die erfindungsgemäße Lösung wird einerseits ermöglicht, daß eine Vorrichtung eingesetzt werden kann, mit der Ureterotomieschnitte an verschiedenen Stellen des Harnleiters offengehalten werden kann, ohne daß die erfindungsgemäße Vorrichtung die Eigenperistaltik und damit die Drainagewirkung der Niere hindert.

In bevorzugter Ausgestaltung ist vorgesehen, daß die Ureterotomieschiene distal eine Verdickung aufweist. Hierdurch wird ein Abrutschen des Aufweitkörpers beim Herausziehen der erfingungsgemäßen Vorrichtung aus den Harnleiter zuverlässig verhindert. Bei bekannten Ureterotomieschienen sind Abbiegungen des distalen Endes bekannt. In weiterer bevorzugter Ausgestaltung sieht die Erfindung vor, daß die Ureterotomieschiene sowohl distal als auch proximal hakenförmig abgebogene Enden aufweist.

Ureterotomieschiene und Aufweitkörper können aus geeigneten Kunststoffmaterialien, wie Polyurethan, Polyethylen, vorzugsweise Silikon oder Gemischen derselben bestehen. Sie können röntgendicht oder transparent sein. Zur Röntgendichtigkeit werden geeignete Metallsalze dem Kunststoff beigemischt.

Während das Verhältnis von Länge des Aufweitkörpers zur Länge der Ureterotomieschiene bzw. deren gestreckten Tiel in weiten Bereichen gewählt werden kann, ist in bevorzugter Ausgestaltung vorgesehen, daß die Länge des Aufweitkörpers nicht mehr als die Hälfte der gestreckten Länge der Ureterotomieschiene beträgt. In weiterer bevorzugter Ausgestaltung ist das Verhältnis von Aufweitkörper zur gestreckter Länge der Schiene maximal ein Drittel da sich herausgestellt hat, daß die Länge von Stenosen dabei zuverlässig abgedeckt werden kann. Bei Schienenlängen von 16 bis 32 cm und vorzugsweise in der Größenordnung von 28 cm beträgt die Länge des Ausweitkörpers zwischen 2 und 15 cm. Auch die Stärke sowohl der Schiene als auch des Aufweitkörpers kann in weiten Bereichen variieren. Bevorzugte Stärken für die Schiene sind etwa 4 bis 9 charrier (1 charrier entspricht 0,33 mm), während die Stärke des Aufweitkörpers vorzugsweise zwischen 7 bis 20 charrier liegt. In äußerster bevorzugter Ausgestaltung ist die Stärke des Aufweitkörpers etwa doppel bis dreifach so stark wie die Stärke der Schiene.

Weitere Vorteile und Merkmale der Erfindung ergeben sich aus den Ansprüchen und aus der nachfolgenden Beschreibung, in der ein Ausführungsbeispiel der erfindungsgemäßen Vorrichtung unter Bezugnahme auf die Zeichnung im einzelnen erläutert ist. Dabei zeigt:

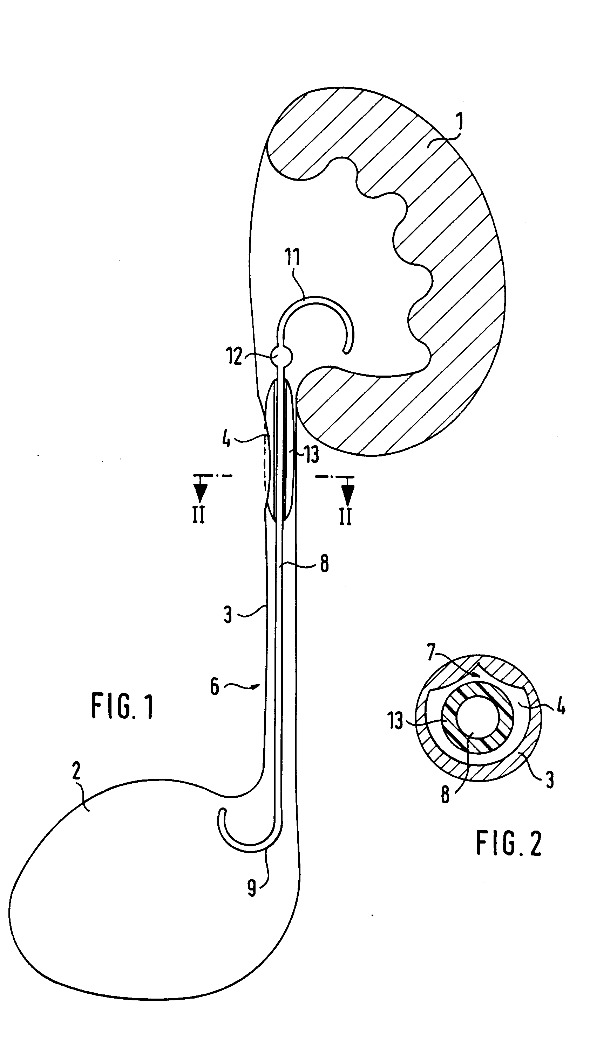

Fig. 1 eine erfindungsgemäße Vorrichtung in Seitenansicht (im eingesetzten Zustand); und

Fig. 2 einen Querschnitt entsprechend II-II der Figur.

Mit 1 ist eine Niere bezeichnet, mit 2 die Blase und mit 3 der Niere 1 und Blase 2 berbindende Harnleiter. Bei 4 weist der Harnleiter 3 eine durch Ureterotomie behobene Stenose auf. Im Harnleiter 3 ist die erfindungsgemäße Vorrichtung 6 zum Offenhalten des Ureterotomieschnitts 7 angeordnet. Die erfindungsgemäße Vorrichtung 6 weist eine Ureterotomieschiene 8 auf, die an ihren beiden Enden mit J-förmigen Haken 9, 11 versehen ist. Vor dem distalen Haken 11 ist die Schiene 8 mit einer Verdickung 12 versehen. Auf der Schiene 8 ist koaxial zu dieser ein Aufweitkörper 13 aufgesetzt, der auf der Schiene 8 reibschlüssig verschiebbar ist. Der Aufweitkörper 13 weist bei einer bevorzugten Schiene mit 28 cm Länge etwa eine Länge von 5 cm auf. Während die Schiene eine Stärke von 5 charrier (1 charrier = 0,33 mm) aufweist, ist der Aufweitkörper etwa doppelt so dick. Durch den erfindungsgemäß vorgesehenen auf der Schiene 8 gleitbaren Aufweitkörper 13 kann eine erfindungsgemäße Schiene vorgegebener Spezifikation eingesetzt werden, um Ureterotomieschnitte 7 an verschiedenen Stellen des Harnleiters offen zu halten.

**Patentansprüche**

1. Vorrichtung zum Offenhalten eines Ureterotomieschnitts im Harnleiter, mit einer im wesentlichen gestreckten Ureterotomieschiene, dadurch gekennzeichnet, daß auf der Ureterotomieschiene (8) ein Aufweitkörper (13) verschiebbar aufsitzt.

2. Vorrichtung nach Anspruch 1, dadurch gekennzeichnet, daß die Ureterotomieschiene (8) distal eine Verdickung (12) aufweist.

3. Vorrichtung nach Anspruch 1 oder 2, dadurch gekennzeichnet, daß die Ureterotomieschiene (8) sowohl distal als auch proximal hakenförmig abgebogene Enden (9, 11) aufweist.

**Claims**

1. Apparatus for keeping open a ureterotomy incision in the ureter, with a substantially stretched ureterotomy rail, characterized in that a widening body (13) is displaceably mounted on the ureterotomy rail (8).

2. Apparatus according to claim 1, characterized in that at the distal end the ureterotomy rail (8) has a thickened portion (12).

3. Apparatus according to claims 1 or 2, characterized in that the ureterotomy rail (8) has hook-like bent ends (9, 11) at both the distal and proximal ends.

**Revendications**

1. Dispositif pour maintenir ouverte une incision d'urétérotomie dans l'uretère, comprenant un rail d'urétérotomie sensiblement allongé, **caractérisé en ce** qu'un écarteur (13) est monté de manière mobile sur le rail d'urétérotomie (8).

2. Dispositif selon la revendication 1, caractérisé en ce que le rail d'urétérotomie (8) présente un gonflement (12) dans la partie distale.

3. Dispositif selon l'une des revendications 1 ou 2, caractérisé en ce que le rail d'urétérotomie (8) présente, dans la partie distale aussi bien que dans la partie proximale des extrémités (9, 11) courbées en crochets.

FIG. 1

FIG. 2